# EUROPEAN PATENT SPECIFICATION

(11) **EP 1 854 419 B1**
(45) Date of publication and mention of the grant of the patent: **07.01.2009**
(21) Application number: 07251961.4
(22) Date of filing: 11.05.2007
(51) Int. Cl.: A61B 17/28, A61B 17/32

(54) **Box joint medical tool apparatus and method of manufacture**
Chirurgisches Instrument mit Kastengelenk und Verfahren zu dessen Herstellung
Instrument de chirurgie avec articulation à emboîtement et sa méthode de production

(30) Priority: 11.05.2006 US 431481
(43) Date of publication of application: 14.11.2007
(73) Proprietor: Teleflex Medical Incorporated, Limerick, PA 19468-1699 (US)
(72) Inventor: Sexton, Anthony J., Racine, WI 53405-4857 (US)
(74) Representative: Hedges, Martin Nicholas

(56) References cited:
- US-A- 3 911 766
- US-A- 5 133 724

## Description

### FIELD OF THE INVENTION

The present invention relates generally to medical devices. More particularly, the present invention relates to a method of manufacturing a medical instrument having a box joint, and an apparatus corresponding thereto.

### BACKGROUND OF THE INVENTION

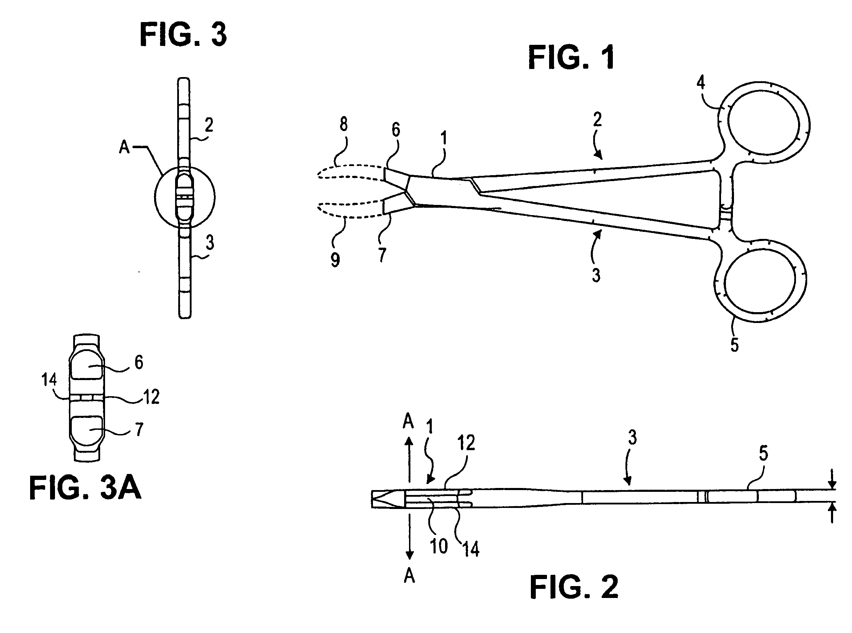

Box joints are a commonly employed coupling and hinge mechanism used in a variety of tools. Included among these tools are various medical instruments, such as a forceps, scissors, or a rongeur. An example of a box joint used in a medical instrument such as a forceps is shown in FIG. 1, which depicts a box joint 1 which couples the handle portions 2,3 of two body parts 4,5 of a forceps (the distal operating end portions opposite the handle portions not being shown). In a box joint configuration, one body part or half of the device, commonly referred to as the "female" portion, includes a channel 10 that is part of the box joint portion mechanism, as shown in FIG. 2. The channel 10 is sized to be just wide enough to accommodate the neck of a complementary male body part or other half of the device, where such neck is arranged to be disposed through the channel 10 so that the two body parts cooperate in a hinge-like manner through the box joint mechanism, as is well known to those skilled in the art.

The general approach to manufacturing such a box jointed forceps is to first forge the left and right blanks which will form the two body parts of the device. The blanks are then machined to size and shape, as dictated by the particular tool to be manufactured. However this method of manufacture and assembly can be particularly difficult for and problematic for box joint type instruments. According to the known prior art, and with reference to the example shown in FIG. 2, the only way to assemble and manufacture a box joint type instrument is to heat the female portion 5 with a suitable heat source, such as, for example, a propane torch, and to then spread the walls 12 and 14 forming the pivot area around channel 10 in the direction of arrows A, and to then insert the male portion 2 through the opening 10 and then hammer the walls 12 and 14 of the female portion 3 back into shape. However this part of the assembly process has to done early in the manufacturing cycle, which means that when the finished instrument needs to be cleaned and polished it is impossible to clean and polish down in the box joint pivot area 1.

Accordingly, it is desirable to provide an improved method of forming, assembling, and manufacturing a medical instrument having a box joint. It is further desirable that the improved method of making a box joint in a medical instrument reduces the costs of manufacture and improves the overall efficiency of the manufacturing process for the medical instrument. It is further desirable to provide an improved method of manufacturing, assembling and finishing a medical device having a box joint where said method will eliminate the need for early assembly of the box joint as elaborated above and will instead allow for better cleaning and polishing of the male and female pivot areas around the box joint portion prior to final assembly of the device.

US-A-5133724 discloses an abdominal aortic clamp according to the preamble of claim 1 which includes a box junction for connecting the opposing body halves of a scissor type tool. This document does not in itself provide any detailed disclosure of the nature of the box junction but refers to the disclosure of US 3952724, which teaches only a conventional type box junction which would, therefore, require assembly by spreading the walls of the female box junction so as to enable the end of the body half carrying the male box joint part, remote from the handle, to pass through the female box joint. This, however, is a conventional type arrangement as discussed previously which therefore suffers from the same problems already discussed above.

The foregoing needs are met, to a great extent, by the present invention, wherein in one aspect an apparatus is provided that in some embodiments provides an improved method of manufacturing, assembling and finishing a medical device having a box joint where said method will eliminate the need for early assembly of the box joint and will instead allow for better cleaning and polishing of the male and female pivot areas around the box joint portion prior to final assembly of the device.

According to the present invention there is provided a medical instrument, comprising: a first half-body and a second half-body joined by a box joint, the first half-body having a female box joint portion defining a channel for accommodating a male box joint portion in the second half body, the second half-body being formed by at least two pieces including a first piece and a second piece, characterised in that: each piece has a complementary mating means for rigidly coupling the two pieces together, the first piece incorporating the male box joint portion, and in that the region of the first piece encompassing the male box joint portion, the mating means and the region therebetween is sized to fit through the channel of the female box joint portion, wherein the instrument is formed by inserting the male box joint portion and mating means of the first piece of the second half-body through the channel in the first half-body and then rigidly joining the first and second pieces of the second half-body by permanently coupling the complementary mating means on the first and second pieces.

The present invention further provides a method of assembling a medical instrument having a box joint, comprising: forming the parts of a medical instrument according to the invention; inserting the male box joint portion through the channel in the female box joint portion; and coupling the complementary mating means of the second half-body to join the first and second pieces to form the second half-body.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In order that the invention may be well understood, there will now be described an embodiment thereof, given by way of example, reference being made to the accompanying drawings, in which:
FIG. 1 is a plan view showing a medical instrument with a box joint;
FIG. 2 is a bottom view showing the female portion of the medical instrument illustrated in FIG 1;
FIG. 3 is a front view of the medical instrument shown in FIG. 1;
FIG. 3A is an enlarged front view of the area A around the box joint of the medical instrument shown in FIG. 3;
FIG. 4 is a perspective view of a female portion of a medical instrument in one embodiment of the present invention; and
FIG. 5 is a perspective view of two portions which form the male portion of the medical instrument according to the present invention.

### DETAILED DESCRIPTION

The invention will now be described with reference to the drawing figures, in which like reference numerals refer to like parts throughout. An embodiment in accordance with the present invention provides a medical forceps having an improved method of manufacture thereof. The method of the present invention includes a method of forming, assembling and manufacturing a medical tool, such as a medical forceps, which has a box joint, the method eliminating the need for initial assembly of the box joint through spreading apart the sidewalls of the female box joint portion. The present invention thereby allows for better cleaning and polishing of the male and female pivot areas around the box joint portion prior to final assembly of the device.

Turning now to the figures, FIG. 1 shows a plan view of a medical instrument, such as a forceps, having a box joint. Such a box joint is well-known to those of ordinary skill in the art. The box joint 1 shown in FIG. 1 joins two pieces of the tool, which are referred to herein as a "half-body." The tool can include a first "male" half-body 2 and a second "female" half-body 3, which are conjoined at the box joint portion 1. Each of the male and female half-bodies 2 and 3 can have handle portions 4 and 5 at their proximal ends. In accordance with conventional practice, as used herein, the term "proximal" or "proximal end" shall refer to the specified end of a device or its component which is generally closer to the medical personnel handling or manipulating the device as it is intended to be used, and the term "distal" or "distal end" shall refer to the specified end of a device or its component which is opposite the proximal end. The medical instrument therefore includes distal first and second operating end portions 6 and 7. Each of the first and second operating end portions 6 and 7 can have any of a variety of well-known distal extensions 8 and 9, respectively, which are shown in FIG. 1 as dotted lines so that the scope of the present invention is understood to include any medical instrument having any distal operating end, such as, for example, a forceps.

FIG. 2 is a top view showing the female portion of the medical instrument illustrated in FIG 1. The box joint portion 1 is shown with the male half-body's neck portion 10 which fits between the two sidewalls 12 and 14 that make up the female portion of the box joint defined by the female half-body 3. The two sidewalls 12 and 14 therefore define a channel into which the male neck portion 10 fits to produce a snug box joint interconnection. Conventional assembly of such a box joint involves the initial spreading apart of the two sidewalls 12 and 14 in the direction of arrows "A" shown in FIG. 2, after which the male half-body 2 is then inserted between the two sidewalls 12 and 14 which are then reformed and straightened to close the box joint. FIG. 3 is a front view of the medical instrument shown in FIG. 1. FIG. 3A is an enlarged front view of the area A around the box joint of the medical instrument shown in FIG. 3.

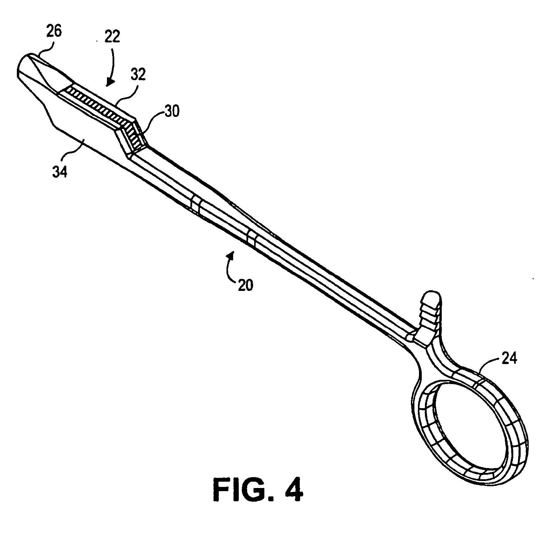

The present invention provides a method of assembly and resulting medical tool having a box joint, whereby the conventional step of spreading apart the box joint's female wall portions is obviated by the inventive principles disclosed herein. FIG. 4 is a perspective view of a female portion or half-body 20 of a medical instrument in one embodiment of the present invention. The female half-body 20 includes a female box joint portion 22 interposed between a proximal handle portion 24 and a distal operating end portion 26 (extreme distal tip not shown). A channel 30 is shown between the two sidewalls 32 and 34 of the female box joint portion 22.

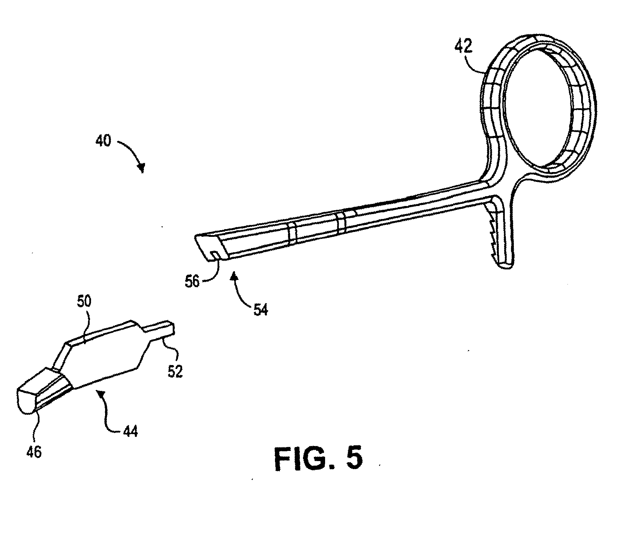

FIG. 5 is a perspective view of two portions which form the male portion or second half-body 40 of the medical instrument according to the present invention. The second half-body 40 includes two pieces, a second proximal handle portion 42 of the medical instrument, and a male box joint portion 44 having a distal operating end portion 46 (extreme distal tip not shown). The male box joint portion 44 includes a neck portion 50 which can generally be a narrow, thin planar element configured to snugly fit within the channel 30 defined by the female box joint portion 22. As such, the male box joint portion 44 is complementary to the female box joint portion 22 so as to form a properly interconnected and operating box joint for the medical instrument.

The male box joint portion 44 also includes a first mating end portion 52. Mating end 52 is shown in the embodiment in FIG. 5 as a finger-like extension or protrusion on the proximal end of the male box joint portion 44, extending from the planar neck portion 50. Mating end 52 is configured and shaped to fit into a corresponding and complementary second mating end 54 defined at the distal end of the handle portion 42 of the male half-body 40. Complementary second mating end 54 is defined in the embodiment shown in FIG. 5 as a narrow slot 56 into which the finger-like protrusion 52 can fit. Interconnection of the two complementary mating ends 52 and 54 can thereby couple the two pieces 42 and 44 of the male half-body.

According to the present invention, a medical device having a box joint is assembled by first making or machining the male portion 40 of the instrument in two pieces 42 and 44, such as shown in FIG. 5, as well as the female half-body 20 as shown in FIG. 4. The surface areas of both the male and female pieces and elements can be cleaned and polished properly and easily before assembly, especially around the critical box joint portions. The instrument can then be assembled by sliding the male box joint portion 44 through the channel 30 of the female box joint portion 22. Then the slot 56 in the male handle portion 42 can be aligned to the corresponding protrusion 52 on the male box joint portion 44, and both parts 42 and 44 can be permanently coupled together. Such coupling can be performed in a myriad of ways, but, in a preferred embodiment, can be accomplished by resistance welding. Resistance welding can be much stronger than laser welding or some other form of welding, because the three surfaces on the protrusion 52 which are in apposition with the corresponding surfaces of slot 56 will be completely welded together providing a stronger bonded joint. The method of the present invention therefore would allow for very intricate distal tips on the instrument to be precision investment cast, regardless of the volumes, at a very cost effective rate. It is understood that the complementary mating means made up of elements 52 and 54,56 of the embodiment shown in FIG. 5 are but one form of mating and coupling the two pieces 42 and 44 of the male half-body 40, and that any number of alternative mating means can be incorporated into the present invention.

## Claims

1. A medical instrument, comprising:
a first half-body (20) and a second half-body (40) joined by a box joint (22,44),
the first half-body (20) having a female box joint portion (22) defining a channel (30) for accommodating a male box joint portion (44) in the second half body (40),
the second half-body (40) being formed by at least two pieces (42,46) including a first piece (46) and a second piece (42),
**characterised in that:** each piece (42,46) of said second half-body (40); has a complementary mating means (52,54,56) for rigidly coupling the two pieces together, the first piece (46) incorporating the male box joint portion (44), and **in that** the region of the first piece (46) encompassing the male box joint portion (44), the mating means (52) and the region therebetween is sized to fit through the channel (30) of the female box joint portion (22), wherein the instrument is formed by inserting the male box joint portion (44) and mating means (52) of the first piece (46) of the second half-body through the channel (30) in the first half body (20) and then rigidly joining the first (46) and second (42) pieces of the second half-body (40) by permanently coupling the complementary mating means (52,54,56) on the first and second pieces (46,42).

2. A medical instrument of claim 1, wherein the mating means (52,54,56) on one of the at least two pieces (42,46) includes an elongate protruding element (52), the elongate protruding element (52) being shaped to fit into a corresponding slot (56) defined by the mating means in another of the at least two pieces (44).

3. A medical instrument of claim 1 or claim 2, wherein the first (46) and second (42) pieces of the second half-body are joined by permanently coupling the complementary mating means on the first and second pieces by resistance welding.

4. A medical instrument according to any of the preceding claims, wherein the first mating means comprises an elongate protrusion adapted to fit into a corresponding slot defined by the second mating means in the second handle portion.

5. A medical instrument according to any of the preceding claims, wherein the medical instrument is a forceps.

6. A method of assembling a medical instrument having a box joint (1), comprising:
forming the parts of a medical instrument according to any of the preceding claims;
inserting the male box joint portion (44) through the channel (30) in the female box joint portion (22); and
coupling the complementary mating means (52, 54, 56) of the second half-body (40) to join the first and second pieces (46,42) to form the second half-body (40).

7. A method of claim 6, wherein the step of coupling the first complementary mating means (52,54,56) to join the first (46) and second (42) pieces of the second half-body (40) comprises resistance welding.

## Patentansprüche

1. Medizinisches Instrument, das umfasst:
einen ersten Halbkörper (20) und einen zweiten Halbkörper (40), die durch ein Kastengelenk (box joint) (22, 44) verbunden sind,
wobei der erste Halbköper (20) einen Aufnahme-Kastengelenkabschnitt (22) hat, der eine Rinne (30) zum Aufnehmen eines Einführ-Kastengelenkabschnitts (44) in dem zweiten Halbkörper (40) aufweist,
und der zweite Halbkörper (40) durch wenigstens zwei Einzelteile (42, 46) gebildet wird, die ein erstes Einzelteil (46) sowie ein zweites Einzelteil (42) enthalten,
**dadurch gekennzeichnet, dass:**
jedes Einzelteil (42, 46) des zweiten Halbkörpers (40) eine komplementäre Eingriffseinrichtung (42, 54, 56) zum starren Verbinden der zwei Einzelteile miteinander hat, das erste Einzelteil (46) den Einführ-Kastengelenkabschnitt (44) enthält, und dass der Bereich des ersten Einzelteils (46), der den Einführ-Kastengelenkabschnitt (44), die Eingriffseinrichtung (52) und den Bereich dazwischen umschließt, so bemessen ist, dass er durch die Rinne (30) des Aufnahme-Kastengelenkabschnitts (22) hindurch passt, wobei das Instrument ausgebildet wird, indem der Einführ-Kastengelenkab-schnitt (44) und die Eingriffseinrichtung (52) des ersten Einzelteils (46) des zweiten Halbkörpers durch den Kanal (30) in dem ersten Halbkörper (20) eingeführt werden und dann das erste (46) und das zweite (42) Einzelteil des zweiten Halbkörpers (40) starr zusammengefügt werden, indem die komplementären Eingriffseinrichtungen (52, 54, 56) an dem ersten und dem zweiten Einzelteil (46, 42) fest verbunden werden.

2. Medizinisches Instrument nach Anspruch 1, wobei die Eingriffseinrichtungen (52, 54, 56) an einem der wenigstens zwei Einzeleile (42, 46) ein längliches vorstehendes Element (52) enthalten und das längliche vorstehende Element (52) so geformt ist, dass es in einen entsprechenden Schlitz (56) passt, den die Eingriffseinrichtung in einem anderen der wenigstens zwei Einzelteile (44) aufweist.

3. Medizinisches Instrument nach Anspruch 1 oder 2, wobei das erste (46) und das zweite (42) Einzelteil des zweiten Halbkörpers zusammengefügt werden, indem die komplementären Eingriffseinrichtungen an dem ersten und dem zweiten Einzelteil durch Widerstandsschweißen fest verbunden werden.

4. Medizinisches Instrument nach einem der vorangehenden Ansprüche, wobei die erste Eingriffseinrichtung einen länglichen Vorsprung umfasst, der so eingerichtet ist, dass er in einen entsprechenden Schlitz passt, der durch die zweite Eingriffseinrichtung in dem zweiten Griffabschnitt gebildet wird.

5. Medizinisches Instrument nach einem der vorangehenden Ansprüche, wobei das medizinische Instrument eine Zange ist.

6. Verfahren zum Zusammensetzen eines medizinischen Instrumentes mit einem Kastengelenk (1), das umfasst:
Ausbilden der Einzelteile eines medizinischen Instruments nach einem der vorangehenden Ansprüche;
Einführen des Einführ-Kastengelenkabschnitts (44) durch die Rinne (33) in dem Aufnahme-Kastengelenkabschnitt (22); und
Verbinden der komplementären Eingriffseinrichtungen (52, 54, 56) des zweiten Halbkörpers (40), um das erste und das zweite Einzelteil (46, 42) zusammenzufügen und den zweiten Halbkörper (40) auszubilden.

7. Verfahren nach Anspruch 6, wobei der Schritt des Verbindens der ersten komplementären Eingriffseinrichtungen (52, 54, 56) zum Zusammenfügen des ersten (46) und des zweiten (42) Einzelteils des zweiten Halbkörpers (40) Widerstandsschweißen umfasst.

## Revendications

1. Instrument médical, comprenant :
un premier demi-corps (20) et un second demi-corps (40) reliés ensemble par un joint introduit (22, 44),
le premier demi-corps (20) ayant une partie de joint introduit femelle (22) définissant un canal (30) destiné à recevoir une partie de joint introduit mâle (44) dans le second demi-corps (40),
le second demi-corps (40) étant constitué d'au moins deux parties (42, 46) comportant une première partie (46) et une seconde partie (42),
**caractérisé en ce que** chaque partie (42, 46) dudit second demi-corps (40) a des moyens d'accouplement complémentaires (52, 54, 56) destinés à coupler les deux parties ensemble de manière rigide, la première partie (46) incorporant la partie de joint à emboîtement mâle (44), et **en ce que** la région de la première partie (46) comprenant la partie de joint introduit mâle (44), les moyens d'accouplement (52) et la zone situé entre ceux-ci, est dimensionnée pour s'agencer à travers le canal (30) de la partie de joint introduit femelle (22), de sorte que l'instrument est formé en insérant la partie de joint introduit mâle (44) et les moyens d'accouplement (52) de la première partie (46) du second demi-corps à travers le canal (30) jusque dans le premier demi-corps (20), et en reliant ensuite de manière rigide les première (46) et seconde (42) parties du second demi-corps (40) en couplant de manière permanente les moyens d'accouplement complémentaires (52, 54, 56) sur les première et seconde parties (46, 42).

2. Instrument médical selon la revendication 1, dans lequel les moyens d'accouplement (52, 54, 56) sur l'une des au moins deux parties (42, 46) comportent un élément allongé faisant saillie (52), l'élément allongé faisant saillie (52) étant mis en forme pour s'agencer dans une fente correspondante (46) définie par les moyens d'accouplement dans l'autre des au moins deux parties (44).

3. Instrument médical selon la revendication 1 ou 2, dans lequel les première (46) et seconde (42) parties du second demi-corps sont reliées en couplant de manière permanente les moyens de couplage complémentaires sur les première et seconde parties par soudage par résistance.

4. Instrument médical selon l'une quelconque des revendications précédentes, dans lequel les premiers moyens d'accouplement comportent une saillie allongée adaptée pour se loger dans une fente correspondante définie par les seconds moyens d'accouplement dans la seconde partie de poignée.

5. Instrument médical selon l'une quelconque des revendications précédentes, dans lequel l'instrument médical est un forceps.

6. Procédé d'assemblage d'un instrument médical ayant un joint introduit (1), comportant les étapes consistant à :
former les parties d'un instrument médical selon l'une quelconque des revendications précédentes,
insérer la partie de joint introduit mâle (44) à travers le canal (30) dans la partie de joint introduit femelle (22), et
coupler des moyens d'accouplement complémentaires (52, 54, 56) du second demi-corps (40) afin de relier ensemble les première et seconde parties (46, 42) afin de former le second demi-corps (40).

7. Procédé selon la revendication 6, dans lequel l'étape consistant à coupler les premiers moyens d'accouplement complémentaires (52, 54, 56) afin de réunir les première (46) et seconde (42) parties du second demi-corps (40) comporte un soudage par résistance.
